# EUROPEAN PATENT SPECIFICATION

(11) **EP 0 750 911 B1**
(45) Date of publication and mention of the grant of the patent: **10.09.2003**
(21) Application number: 96109035.4
(22) Date of filing: 05.06.1996
(51) Int. Cl.: A61K 33/04, A61K 31/095, A61K 31/66, A61P 35/00

(54) **Composition containing selenium to reduce cancer incidence and extend lifespan**
Selen enthaltende Zusammensetzung zur Reduzierung der Krebshäufigkeit und zur Verlängerung des Lebensalters
Composition contenant du sélénium pour la réduction de la fréquence des cancers et pour l'allongement de la durée de vie

(30) Priority: 07.06.1995 US 911
(43) Date of publication of application: 02.01.1997
(73) Proprietor: Life Science Labs, Inc., Minneapolis, MN 55423 (US)
(72) Inventor: Olson, David M., Minneapolis, Minnesota 55423 (US); Passwater, Richard A., Berlin, MD 21811 (US)
(74) Representative: Müller-Boré & Partner Patentanwälte

(56) References cited:
- DE-A- 3 542 309
- US-A- 5 230 998
- PATENT ABSTRACTS OF JAPAN vol. 016, no. 273 (C-0953), 18 June 1992 & JP-A-04 066567 (KENJI SODA;OTHERS: 01), 2 March 1992,
- J. LABELLED COMPD RADIOPHARM. , vol. 30, 1991, page 143 XP000579305 ENGELSKIRCHEN G. ET AL.: "N.C.A. selenium-73-labeled compounds basic studies on a selenation strategy via triphenylphosphine selenide using N.C.A. selenium-75"

## Description

The present invention relates to a composition comprising a compound including the element Se as well as the use of said compound for preventing or reducing cancer.

The many chemicals that cause cancer as carcinogens or radiation or predisposed genetic flows do so by causing DNA damage by adding oxygen molecules where the oxygen should not be. To the degree that any other of a few chemicals prevent that DNA damage, the chemicals also prevent cancer. Since not all the damage can be prevented from occurring, the most important step is the repair of this damage.

DE-A1-35 42 309 describes a medical antioxidant comprising *inter alia* selenomethionine. The abstract of Japanese Patent JP-A-4066567 describes a medicine including an active ingredient of active oxygen production suppressing agent comprising a selenoglutathione derivative and/or a dimer thereof. Engelskirchen et al. (J. Labelled Compd. Radiopharm., 1991, 30: 143) describes the use of a radiolabelled compound as intermediate reagent for the synthesis of radiopharmaceuticals including a selenation strategy via triphenylphosphine selenide using selenium-75. US-A-5,230,998 discloses *inter alia* chemical tests for the inhibitory activity on the reaction between HIV-1 GP 120 and antibodies specific for the V3 hypervariable loop, wherein for example triphenylphosphine sulfide and oxide have been tested.

Thus, the technical problem underlying the present invention is to provide a new composition for preventing and/or repairing DNA damage caused by oxygen and oxygen-containing molecules, respectively.

The solution to the above technical problem is achieved by providing a composition comprising a compound ("chemical") including the element Se, for preventing or reducing a cancer, wherein the expression "element Se" means all forms of selenium containing compounds, selected from the group comprising -Se-Se-Se- (triselenide), N = C = Se (isoselenocyanate), R₃P=Se (phosphine selenide), and R₂C=Se, where R is any radical. Additionally, this term covers also forms which will be converted within the body into the above effective forms before being eliminated. As a preferred embodiment of the present invention, the compound including the element Se is triphenyl phosphine selenide. In the following, examples of the compound including the element Se are listed:

The triselenide structure -Se-Se-Se- covers CH₂ = CH-CH₂-Se-Se-Se-CH₂-CH = CH₂ diallyl triselenide, the chemicals which constitute most of the Se in garlic.

Selenide also covers CH₂ = CH-CH₂-N = C = Se allyl isoselenocyanate.

R₂C = Se covers CH₃-CH = Se ethylidene selenide and 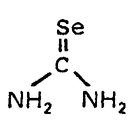 selenourea and other similar structures where R does not contain a carbon atom, but is organic because of the bond to carbon.

The form of chemical in which selenium is present varies the efficacy over a very wide range. Elemental selenium passes through the body after ingestion without becoming biologically available. More preferred forms of selenium will be taken into the bloodstream rather than eliminated from the body quickly as selenonium ion in the urine or exhaled through the lungs as dimethyl selenide. Some forms of selenium are not eliminated, but become stored in tissues where they are less effective than in the bloodstream.

Any double bond within DNA (or messenger RNA and transfer RNA) can be attacked by a chemical carcinogen, free radical, or radiation to form an epoxide (C=C becomes 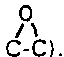 Any 2 epoxides on base pairs can form a crosslink 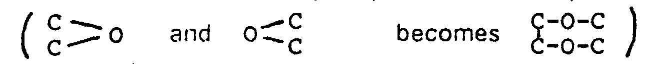 as proposed in United States Application 05/271,655 filed July 14, 1972 (which corresponds to DE-OS 2336176, and claimed that some forms of Se prevented and reversed those crosslinks in some way). This strong crosslink bond then replaces the weak hydrogen bonding that holds base pairs together in DNA. In effect, the zipper of DNA no longer unzips due to the oxygen crosslink, so causes replication to stutter and form random uncontrolled division called cancer. These 2 steps are what the United States National Cancer Institute (NCI) now calls initiation (epoxide formation) and propagation (crosslinking). Many antioxidants help the body keep the epoxide from forming initially. Selenium in the form of e.g., triphenyl phosphine selenide actually attacks the epoxide and reverts it back to a double bond without loss of configuration of the remaining base pair 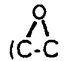 reverts to C=C). The mechanism is:

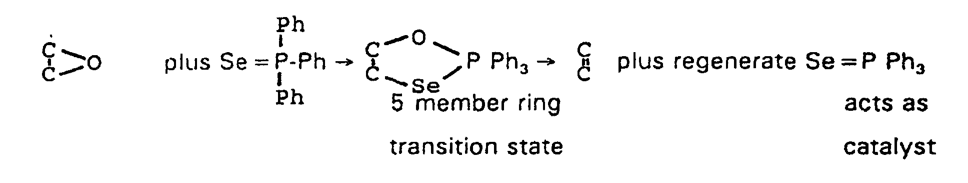

The oxidized forms of selenium can be biologically reduced by vitamin C to the useless elemental form of selenium. The preferred form is the most reduced minus 2 oxidation state of the selenides. Within this class, the best form is that which reacts with epoxides to yield an olefin without change in configuration. Selenides have the general structure -Se- and diselenides -Se-Se- and triselenides -Se-Se-Se- and even better is R₂C=Se. The best form has the structure R₃P=Se where R is any organic radical. An example is Ph₃P=Se, triphenyl phosphine selenide.

In a further embodiment of the present invention, the above defined composition comprises additionally as component vitamin E or a derivative thereof. Vitamin E has a synergistic relationship with selenium such that the two together are almost always better than the best by itself.

In a further embodiment of the present invention, the above defined composition comprises additionally as component a sulphur amino acid such as cysteine, or a derivative thereof, such as glutathione.

In a further embodiment of the present invention, the above defined composition comprises additionally as component a synthetic antioxidant which is edible without toxicity in humans such as tert.-butyl methoxyphenole (BHA), 2,6-di-tert.-butyl-4-methyl phenole (BHT) or ethoxyquin. The preferred synthetic ant-oxidant is BHA.

In a preferred embodiment of the present invention, the relative value of each of the 4 above components - i.e. a compound including the element Se, vitamin E, a sulphur amino acid, and a synthetic antioxidant - is such that selenium is much greater than vitamin E and the sulfur amino acid and synthetic antioxidant are about equal to each other and to vitamin E. It is therefore possible to get most of the effect by using a selenium with high efficacy like triphenyl phosphine selenide alone without the other 3 components. The maximum effect is by using all 4 components and a biological synergism is achieved whereby adding any one is better than it would be without adding the additional one.

The level of carcinogen exposure is different for different people and can even be different for the same person when diet, environment and genetic predisposition are considered. For maximum benefit to a whole population, there must be a minimum that everyone needs and a maximum that can be given and knowingly exceeding the minimum need without causing problems in those who have only minimum need. With selenium, for some forms this range is very narrow.

With the other 3 ingredients, this range is much wider. The ranges and optimum of a preferred composition of the present invention for each of the 4 components daily is:
1) 400 µg of selenium in the form of e.g. triphenyl phosphine selenide as optimum within a range of 20 µg to 20 mg, and other selenium chemicals to 1 g;
2) 0.4 g Vitamin E as optimum within a range of 50 mg to 1 g;
3) 0.6 g cysteine as optimum within a range of 50 mg to 2 g;
4) 50 mg BHA as optimum within a range of 20 µg to 200 mg.

There is over a 1000 fold difference between the most effective and the least effective forms of Se.

## Claims

1. A composition comprising a compound including the element Se, for preventing or reducing cancer, wherein the compound is selected from triselenides, isoselenocyanates, phosphine selenides and compounds having the structure R₂C=Se, wherein R is any radical.

2. The composition according to claim 1, wherein the compound is triphenyl phosphine selenide.

3. The composition according to claim 1 or 2, further comprising vitamin E or a derivative thereof.

4. The composition according to anyone of claims 1 to 3, further comprising a sulfur amino acid or a derivative thereof.

5. The composition according to claim 4, wherein the sulfur amino acid is cysteine.

6. The composition according to claim 4, wherein the sulfur amino acid derivative is glutathione.

7. The composition according to anyone of claims 1 to 6, further comprising a synthetic antioxidant.

8. The composition according to claim 7, wherein the synthetic antioxidant is tert.-butyl methoxyphenole, 2,6-di-tert.-butyl-4-methylphenole or ethoxyquin.

9. The composition according to anyone of the preceding claims, comprising 20 µg to 200 mg of triphenyl phosphine selenide, 50 mg to 1 g of vitamin E, 50 mg to 2 g cysteine, and 20 µg to 200 mg tert.-butyl methoxyphenole.

10. The composition according to claim 9, comprising 400 µg of Se as triphenyl phosphine selenide, 0.4 g vitamin E, 0.6 g cysteine, and 50 mg tert.-butylmethoxyphenole.

11. Use of a composition as defined in anyone of the preceding claims 1 to 10 for the preparation of a medicament for preventing or reducing cancer.

12. Use of a composition as defined in any one of the preceding claims 1 to 10 by a human diagnosed with any type of cancer for the preparation of a medicament which prevents or retards metastasis or spread of cancer to other sites.

## Patentansprüche

1. Zusammensetzung, umfassend eine Verbindung, die das Element Se einschließt, zur Verhinderung oder Verminderung von Krebs, wobei die Verbindung aus Triseleniden, Isoselenocyanaten, Phosphinseleniden und Verbindungen mit der Struktur R₂C=Se, wobei der Rest R jedweder Rest ist, ausgewählt ist.

2. Zusammensetzung nach Anspruch 1, wobei die Verbindung Triphenylphosphinselenid ist.

3. Zusammensetzung nach Anspruch 1 oder 2, weiter umfassend Vitamin E oder ein Derivat davon.

4. Zusammensetzung nach einem der Ansprüche 1 bis 3, weiter umfassend eine Schwefelaminosäure oder ein Derivat davon.

5. Zusammensetzung nach Anspruch 4, wobei die Schwefelaminosäure Cystein ist.

6. Zusammensetzung nach Anspruch 4, wobei das Schwefelaminosäurederivat Glutathion ist.

7. Zusammensetzung nach einem der Ansprüche 1 bis 6, weiter umfassend ein synthetisches Antioxidans.

8. Zusammensetzung nach Anspruch 7, wobei das synthetische Antioxidans tert.-Butylmethoxyphenol, 2,6-Di-tert.-butyl-4-methylphenol oder Ethoxyquin ist.

9. Zusammensetzung nach einem der vorhergehenden Ansprüche, umfassend 20 µg bis 200 mg Triphenylphosphinselenid, 50 mg bis 1 g Vitamin E, 50 mg bis 2 g Cystein und 20 µg bis 200 mg tert.-Butylmethoxyphenol.

10. Zusammensetzung nach Anspruch 9, umfassend 400 µg Se als Triphenylphosphinselenid, 0,4 g Vitamin E, 0,6 g Cystein und 50 mg tert.-Butylmethoxyphenol.

11. Verwendung einer Zusammensetzung, wie in einem der vorhergehenden Ansprüche 1 bis 10 definiert, zur Herstellung eines Medikaments zur Verhinderung oder Verminderung von Krebs.

12. Verwendung einer Zusammensetzung, wie in einem der vorhergehenden Ansprüche 1 bis 10 definiert, bei einem Menschen, bei dem eine Krebsart diagnostiziert worden ist, zur Herstellung eines Medikaments, welches Metastasen oder das Streuen von Krebs auf andere Stellen verhindert oder verzögert.

## Revendications

1. Composition qui comprend un composé comprenant l'élément Se, pour la prévention ou la réduction du cancer, dans laquelle le composé est choisi entre des triséléniures, des isosélénocyanates, des séléniures de phosphines et des composés de structure R₂C=Se, dans laquelle R représente n'importe quel radical.

2. Composition suivant la revendication 1, dans laquelle le composé consiste en séléniure de triphénylphosphine.

3. Composition suivant la revendication 1 ou 2, comprenant en outre de la vitamine E ou un de ses dérivés.

4. Composition suivant l'une quelconque des revendications 1 à 3, comprenant en outre un amino-acide soufré ou un de ses dérivés.

5. Composition suivant la revendication 4, dans laquelle l'amino-acide soufré est la cystéine.

6. Composition suivant la revendication 4, dans laquelle le dérivé d'amino-acide soufré est le glutathion.

7. Composition suivant l'une quelconque des revendications 1 à 6, comprenant en outre un oxydant synthétique.

8. Composition suivant la revendication 7, dans laquelle l'oxydant synthétique est le tertio-butylméthoxyphénol, le 2,6-di-tertio-butyl-4-méthylphénol ou l'éthoxyquine.

9. Composition suivant l'une quelconque des revendications précédentes, comprenant 20 µg à 200 mg de séléniure de triphénylphosphine, 50 mg à 1 g de vitamine E, 50 mg à 2 g de cystéine et 20 µg à 200 mg de tertiobutylméthoxyphénol.

10. Composition suivant la revendication 9, comprenant 400 µg de Se sous forme de séléniure de triphénylphosphine, 0,4 g de vitamine E, 0,6 g de cystéine et 50 mg de tertiobutylméthoxyphénol.

11. Utilisation d'une composition suivant l'une quelconque des revendications 1 à 10 précédentes pour la préparation d'un médicament destiné à la prévention ou la réduction du cancer.

12. Utilisation d'une composition suivant l'une quelconque des revendications 1 à 10 précédentes, par un patient humain chez lequel a été diagnostiqué n'importe quel type de cancer, pour la préparation d'un médicament qui prévient ou retarde les métastases ou la dissémination du cancer à d'autres sites.
